(19) Europäisches Patentamt
European Patent Office
Office européen des brevets

(11) Publication number: 0 325 270 A2

# (12) EUROPEAN PATENT APPLICATION

(21) Application number: 89100957.3

(22) Date of filing: 20.01.89

(51) Int. Cl.4: A61K 47/00 , A61K 39/44

Claims for the following Contracting State: ES.

(30) Priority: 22.01.88 JP 13089/88

(43) Date of publication of application:
26.07.89 Bulletin 89/30

(84) Designated Contracting States:
BE DE ES FR GB IT NL SE

(71) Applicant: GREEN CROSS CORPORATION
15-1, Imabashi 1-chome Higashi-ku
Osaka-shi
Osaka(JP)

(72) Inventor: Munechika, Koji
3-A58-507, Otokoyama Karo
Yahata-shi Kyoto(JP)
Inventor: Ueda, Yasuo
2-6-4, Furuedai
Suita-shi Osaka(JP)
Inventor: Kikukawa, Akihito
1-19-1, Nagaomotomachi
Hirakata-shi Osaka(JP)
Inventor: Nakae, Takashi
14-7-307, Satsukigaoka Kita
Suita-shi Osaka(JP)
Inventor: Yamanouchi, Kouichi
1-3-14, Shiroyamadai
Sakai-shi Osaka(JP)

(74) Representative: Kolb, Helga, Dr. Dipl.-Chem. et al
Hoffmann, Eitle & Partner Patentanwälte
Arabellastrasse 4
D-8000 München 81(DE)

## (54) Anticancer conjugates.

(57) An anticancer conjugate comprising an anticancer substance coupled to a carrier via aldohexopyranose ring-cleaved dextran wherein the aldohexopyranose ring-cleaved dextran is linked to the carrier through a carboxyl group on an amino acid protecting said dextran and an amino group on said carrier and linked to the anticancer substance through an aldehyde group on said dextran and an amino group on said anticancer substance.

EP 0 325 270 A2

# ANTICANCER CONJUGATES

## FIELD OF THE INVENTION

This invention relates to an anticancer conjugate in which an anticancer substance is coupled to a carrier via aldohexopyranose ring-cleaved dextran (hereinafter referred to as "cleaved dextran") and which has good accumulability in cancer cells.

## BACKGROUND OF THE INVENTION

A number of substances having anticancer activity have been developed as anticancer drugs for use in clinical practice. However, they are nonspecific to cancer cells, i.e. they are toxic to normal cells as well. Therefore, the use of anticancer drugs alone has not been very successful in producing desired therapeutic effects.

As a result, conjugates of an anticancer substance with a carrier, which is a substance having affinity for cancer cells, have been prepared and caused to arrive at cancer cells specifically and exhibit anticancer activity against the cancer cells. For example, conjugates of adriamycin, daunomycin and the like, which have anticancer activity, with antibodies to cancer cell surface antigens linked together through dextran have been developed by E. Hurwitz et al. (Europ, J. Cancer, vol. 14, pages 1213-1220, 1978). The method of producing these conjugates comprises binding the amino group of the carrier and the amino group of the anticancer substance to the aldehyde groups of cleaved dextran.

However, when conjugates are prepared by the above method, excessive binding of the carrier (generally, a protein) to cleaved dextran tends to occur since the cleaved dextran has a number of aldehyde groups. As a result, large molecules may form which decrease in the yield of the desired conjugate and decrease the activity of the carrier.

Also, these conjugates may possibly be contaminated with unreacted raw materials and others (e.g. anticancer substance, anticancer substance-cleaved dextran conjugate) in the process of their production.

On the other hand, it is desired that the anticancer substance-to-carrier binding ratio (i.e. the value of anticancer/carrier) should be increased without decreasing the carrier activity of said conjugates.

Furthermore, it is desired that the anticancer substance-cleaved dextran conjugate should be released from said conjugates as little as possible.

## SUMMARY OF THE INVENTION

Accordingly, it is an object of the invention to provide anticancer conjugates which can be produced from a carrier, an anticancer substance and cleaved dextran in high yields and with good reaction efficiency.

Another object of the invention is to provide anticancer conjugates which can be produced from a carrier, an anticancer substance and cleaved dextran in high yields and with good reaction efficiency.

Another object of the invention is to provide anticancer conjugates which are substantially free of unreacted raw materials and other contaminants and have a high anticancer substance/carrier ratio and from which the anticancer substance-cleaved dextran conjugate can hardly be released.

The present inventors made intensive investigations in an attempt to find out a method of producing carrier-cleaved dextran-anticancer substance conjugates more efficiently and, as a result, found that when about 5 to 70 percent of the aldehyde groups on cleaved dextran, which serves as a spacer, are protected by binding an amino acid thereto and the thus-protected cleaved dextran is reacted with an anticancer substance and a carrier, in other words when the free aldehyde groups of said protected dextran are reacted with an anticancer substance in the manner of Schiff base formation reaction and the free carboxyl groups of the protected cleaved dextran which are originating from the protective group amino acid are bound to the carrier in the manner of peptide bond formation reaction, the product conjugates can achieve the above objects.

Thus, the invention provides an anticancer conjugate comprising aldohexopyranose ring-cleaved dextran, an anticancer substance and a carrier, characterized in that a conjugate of the anticancer substance with the cleaved dextran is linked to the carrier through an amino acid.

## DETAILED DESCRIPTION OF THE INVENTION

In the practice of the invention, the cleaved dextran preferably has a molecular weight of 1,000 to 100,000, more preferably 10,000 to 70,000 and preferably a cleaved aldohexopyranose ring content of 10 to 100 percent, more preferably 50 to 100 percent. The cleaved aldohexopyranose ring moiety may be represented by formula (I) below.

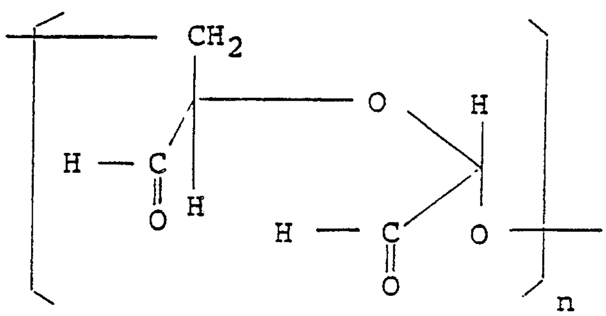

(I)

The cleavage of the aldohexopyranose ring of dextran is generally carried out by oxidation. The use of sodium periodate as the oxidizing agent is preferable. Thus, for instance, cleaved dextran such as mentioned above can be produced by adding, to dextran, an oxidizing agent in an amount of about 80 to 4,000 mg, preferably about 130 to 3,000 mg, more preferably about 130 to 2,640 mg, per gram of dextran and performing the reaction at 4° C to 25° C for about 1 to 24 hours in distilled water. The reaction mixture is then purified, for example, by dialyzing it against distilled water for 10 to 30 hours, preferably followed by lyophilization. The thus-obtained cleaved dextran preferably has a degree of cleavage of glucose residues of about 10 to 100 percent, more preferably about 50 to 100 percent.

Binding of 5 to 70 percent, preferably about 10 to 50 percent, of the aldehyde groups in this cleaved dextran to an amino acid gives amino acid-protected cleaved dextran. This amino acid serves to achieve cleaved dextran-carrier coupling and at the same time protects excess free aldehyde groups.

Specific examples of the amino acid includes the following:

Neutral amino acids:

Aliphatic amino acids (glycine, alanine, valine, leucine, isoleucine, etc.), oxo amino acids (serine, threonine, etc.), sulfur-containing amino acids (cysteine, cystine, methionine, etc.), aromatic amino acids (phenylalanine, tyrosine, tryptophan, etc.);

Acidic amino acids:

Aspartic acid, glutamic acid, etc;

Basic amino acids:

Histidine, lysine, arginine, etc.;

Imino acid residues:

Proline, hydroxyproline, etc.;

Amino acids other than α-amino acids:

β-Alanine, 6-amino-n-caproic acid, etc.

Among these amino acids, glycine, alanine, serine, aspartic acid, β-alanine and 6-amino-n-caproic acid are preferably used.

In the practice of the invention, the protection of the aldehyde groups in the cleaved dextran with an amino acid can be performed, for example, in the following manner. To 10-100% cleaved dextran is added an amino acid (preferably together with a reducing agent such as $NaBH_3CN$) in an amount of about 10 to 200 µmoles per 10 mg of the cleaved dextran, and the reaction is carried out at 20° C to 30° C for 3 to 24 hours in a phosphate buffer (pH 6.0) to give protected cleaved dextran with 5 to 70 percent of the aldehyde groups being protected with the amino acid. On this occasion, each aldehyde group and the amino acid form a Schiff base, which is then reduced to give a secondary or tertiary amine. Since the Schiff base is unstable, the conversion to a secondary or tertiary amine is performed for the purpose of stabilization.

The protected cleaved dextran is reacted with an anticancer substance and a carrier. On that occasion, the reaction order is not critical. It is preferable, however, to first react the protected cleaved dextran with the anticancer substance and then react the thus-obtained intermediate compound with the carrier.

The protected cleaved dextran as such may be treated for its binding to an anticancer substance. When the number of aldehyde groups available for reaction with the anticancer substance is insufficient (for example, when the extent of aldehyde group protection is excessive, or when the extent of dextran cleavage is insufficient), however, the protected cleaved dextran may be subjected again to aldohexopyranose ring cleavage by oxidation or other means to provide it with new aldehyde groups which are required. While the amount of the oxidizing agent such as sodium periodate should be selected suitably depending on the desired degree of oxidation, it is used, for example, in an amount of about 10 to 32 mg per 10 mg of the protected cleaved dextran. After addition of the oxidizing agent, the reaction is carried out at 4° C to 25° C for 1 to 24 hours, and the reaction mixture is then dialyzed against distilled water for 20 to 40 hours and lyophilized to give cleaved dextran preferably with about 5 to 70 percent, more preferably about 10 to 50 percent, of its aldehyde groups protected. It is also possible to obtain the protected cleaved dextran without the step of reoxidation by adjusting the amount of the protective amino acid.

The thus-obtained protected cleaved dextran is preferably reacted with an anticancer substance.

The anticancer substance may be any one which has a group reactive with the aldehyde group (e.g. amino group). When it has no such group, it may be rendered reactive with the aldehyde group, hence usable in the practice of the invention, by reacting it with an appropriate compound. As suitable examples of the anticancer substance, there may be mentioned daunomycin, mitomycin C and adriamycin.

The reaction between protected cleaved dextran and the anticancer substance is preferably carried out to an extent such that about 1 to 15 µmoles (in the case of adriamycin, for instance, about 0.5 to 7 mg) of the anticancer substance is bound to 10 mg of the protected cleaved dextran. In a preferred embodiment, the reaction is carried out by adding, to an organic solvent (e.g. dimethyl sulfoxide) an aqueous solvent (buffer of pH 6 to 9, such as phosphate buffer) or an organic solvent-water mixture, the cleaved dextran to a final concentration of about 0.1 to 5 w/v% and the anticancer substance to a final concentration of about 0.1 to 5 w/v%. The reaction is conducted 20° C to 50° C for 1 to 30 hours.

Thus is obtained a protected dextran-anticancer substance conjugate.

The thus-obtained intermediate conjugate can be purified by a known technique, such as gel filtration, ion exchange adsorption chromatography, etc.

The protected dextran-anticancer substance conjugate is then reacted with a carrier. The carrier is to serve to convey the anticancer substance focally to the site of cancer. Therefore, any substance which has affinity for cancer cells may be used without any particular limitation. Preferred examples are gamma-globulin, cancer-specific polyclonal or monoclonal antibodies, fibronectin, transferrin, fibrinogen and the like proteins.

More specifically, the cancer-specific antibodies, for instance, include, among others, monoclonal antibodies having the four characteristics mentioned below and fragments thereof.

(1) They are produced by hydridomas produced with a low-differentiation gastric adenoma-drived cell line (MKN-45) as an immunogen;

(2) They belong to the Ig class: IgG1 (κ);

(3) They react with the following cancer cells: esophagus cancer cells (TE-3), lung cancer cells (PC-3), stomach cancer cells (MKN-45), stomach cancer cells (KATO-III) and liver cancer cells (HEK).

A method of preparing the cancer-specific antibodies is disclosed in JP-A-61-167699 (the term "JP-A" used herein means "an unexamined published Japanese patent application"), for instance.

The reaction between the protected dextran-anticancer substance conjugate and the carrier can be carried out by any of the methods known for peptide bond formation. Thus, the carboxyl group of the amino acid in the protected cleaved dextran is reacted with an amino group of the carrier.

This reaction is carried out, for example, by the method comprising reacting the carboxyl group in the free acid form with the carrier (direct method) or by the method comprising first converting the carboxyl group to a reactive derivative, such as an active ester or an acid halide (e.g. acid chloride) and then reacting the reactive derivative with the carrier (activation method). This reaction is preferably carried out in the presence of a condensing agent.

In the case of the direct method, a water-soluble carbodiimide, such as 1-ethyl-3-(3-dimethylaminopropyl)-carbodimide, is used as the condensing agent and the reaction is carried out in an aqueous solvent having a pH of 6 to 9 (e.g. phosphate buffer) in a concentration of the intermediate conjugate of about 0.1 to 5 w/v% and a concentration of the carrier of about 0.1 to 5 w/v% at 0 °C to 30 °C for about 1 to 30 hours.

In the case of the activation method (e.g. active ester method), a carbodiimide such as dicyclohexylcarbodiimide is used as the condensing agent, and the intermediate conjugate is first converted to an active ester form by reacting it with an esterifying activation agent, such as N-hydroxylsucciniimide, N-hydroxynorbonene- 2,3-dicarboxyimide or p-nitrophenol, and then reacting the resulting product with the carrier. More specifically, the intermediate conjugate is reacted with the activating agent in an organic solvent (e.g. dimethyl sulfoxide, demethylformamide, dioxane) in an intermediate conjugate concentration of 0.1 to 5 w/v% and an activating agent concentration of 0.1 to 10 w/v% at 0 °C to 30 °C for about 10 minutes to about 30 hours to give an active ester. Then, the intermediate conjugate in the active ester form is reacted with the carrier. In this case, the reaction conditions are, for example, as follows: intermediate conjugate concentration 0.1 to 1 w/v%, carrier concentration 0.01 to 1 w/v% (preferably 0.05 to 0.5 w/V%), solvent: mixed solvent composed of an organic solvent (e.g. dimethyl sulfoxide) and an aqueous solution (e.g. buffer with pH 6 to 9) in a mixing ratio of about 1:9 to 1:1, temperature 0 °C to 30 °C, reaction period about 1 minute to about 10 hours.

For maintaining the specificity (activity) of the carrier, it is preferable to protect the carrier in advance. More specifically, the amino groups of the carrier which are to be retained are protected with an eliminable amino-protecting group, the protected carrier is then reacted with the cleaved dextran-anti-cancer substance conjugate and, after the reaction, the protective group is eliminated.

As such protective group, there may be mentioned dimethylmaleic anhydride, maleic anhydride, citraconic anhydride, ethyl trifluoroacetate and trifuluoroacetic anhydride, among others. The above methods are disclosed, e.g., in Biochem, J., vol. 116, pages 843-849 (1970) and Methods in Enzymol., vol. 11, pages 317-322.

The thus-obtained conjugate according to the invention can be purified by gel filtration, ion exchange chromatography and/or affinity chromatography using an immobilized antigen, for instance.

The conjugate according to the invention has a molecular weight of about 100,000 to 500,000, preferably about 200,000 to 350,000.

The binding ratios of the anticancer substance and amino acid to the dextran in the final conjugate are about 1 to 30 μmoles, preferably about 10 to 20 μmoles, and about 12 to 62 μmoles, preferably about 20 to 40 μmoles, to 10 mg of the dextran, respectively, corresponding to about 1 to 25 percent, preferably about 10 to 15, and about 10 to 50 percent, preferably about 15 to 30 percent, of the aldehyde groups of the 100% cleaved dextran, respectively.

The binding ratio between the anticancer substance and the carrier is about 5:1 to 30:1.

The thus-obtained conjugate according to the invention is made up into a dried product, for example by filter sterilization of the final reaction mixture, distribution of the filtrate into vials or the like and lyophilization of the same.

The conjugate provided by the invention has specific affinity for cancer cells in warm-blooded animals (in particular, mammals), such as human, cattle, horse, rat and mouse, exhibits excellent anticancer activity against cancer in such animals, and is useful as an anticancer agent.

The conjugate according to the invention is administered, for example by intravenous administration or intravenous drip infusion. The conjugate is made up into dosage forms, such as injections or solutions for drip infusion, by using pharmaceutically acceptable excipients, diluents or the like, for example distilled water for injection or physiological saline. The dose of the conjugate according to the invention may vary depending on the species, symptom, body weight, age and other factors of the target of administration. Generally, however, when the anticancer substance is adriamycin, about 10 to 1,500 mg (about 10 to 300 mg as adriamycin) of the conjugate according to the invention is administered daily to a human adult in the form of a solution in 0.5 to 500 ml of an appropriate solvent in a single dose or in several divided doses.

The conjugate according to the invention, in which the carrier (protein) is linked to the cleaved dextran through a peptide bond, can be produced by ordinary amidation. Thus, the conjugate according to the invention can be obtained in high yields since the reactions in the process of its production can be readily controlled and aggregation due to excessive binding of the raw materials hardly occurs (thus causing little contamination by large molecule by-products).

It is an additional advantageous feature of the conjugate according to the invention that the conjugate is hardly contaminated by unreacted materials (for example, anticancer substance, anticancer substance-cleaved dextran conjugate) in the process of its production, has a high anticancer/carrier ratio and hardly allows release of the anticancer substance-cleaved dextran conjugate therefrom.

The conjugate according to the invention is considered to be very useful in targeting therapy of cancer.

The following examples are further illustrative of the present invention but are by no means limitative of the scope of the invention.

## EXAMPLE 1

(1) Oxidation of dextran

Five (5) grams of dextran (molecular weight 70,000) was weighed and dissolved in 450 ml of distilled water. 50 ml of a sodium metaperiodate solution (containing 6.6 g of $NaIO_4$) was added, and the reaction was carried out in a dark place at room temperature for 5 hours, whereby the glucose residues of dextran T-70 (manufactured by Pharmacia Fine Chemicals) were oxidized. The reaction mixture was dialyzed against distilled water for 30 hours and then lyophilized to give 50% cleaved dextran.

(2) Protection with glycine

One (1) gram of this cleaved dextran was weighed and dissolved in 100 ml of 50 mM phosphate buffer (pH 6.0). 695 mg of solid-form glycine was added for Schiff base formation between an aldehyde group of the cleaved dextran and the amino group of glycine, with simultaneous addition of 10 ml of an aqueous solution containing 583 mg of sodium cyanoborohydride. The resultant mixture was stirred at 25° C for 20 hours whereby the Schiff base formed was reduced. The reaction mixture was dialyzed against distilled water for 30 hours. Then, 2.7 g of $NaIO_4$ was added for oxidizing the remaining glucose residues, and the resultant mixture was stirred in a dark place at room temperature for 5 hours. The reaction mixture was dialyzed against distilled water and then lyophilized to give cleaved dextran 50% protected by glycine.

(3) Binding of adriamycin

Forty (40) milligrams of the cleaved dextran (with 50% of its aldehyde groups modified by glycine) obtained in the above step (2) was weighed and dissolved in 500 µg of dimethyl-sulfoxide. To this solution was added 500 µg of a dimethyl-sulfoxide solution containing 10 mg of adriamycin, and the reaction was carried out at 25° C for 20 hours. Thereafter, 2 ml of 50 mM phosphate buffer (pH 7.5) was added to the reaction mixture, and the reaction was again carried out for 3 hours to give an adriamycin-cleaved dextran-glycine conjugate in a crude form. To 3 ml of this crude conjugate solution was added 12 ml of ethanol, and the mixture was stirred and then centrifuged at 9,000 g for 10 minutes. The sediment obtained was washed with two 12-ml portions of ethanol and dried under reduced pressure to give a purified form of the adriamycin-oxidized dextran-glycine conjugate.

(4) Active ester formation

The adriamycin-cleaved dextran-glycine conjugate (500 mg) was dissolved in 15 ml of a 3:1 mixture of dimethyl sulfoxide and dimethylformamido and the solution was cooled with ice. Then, 703.6 mg (3.41 mmoles) of dicyclohexylcarbodiimie was added, the mixture was stirred for 30 minutes, and 428.2 mg (3.72 mmoles) of N-hydroxysuccinimide was further added. The resultant mixture was stirred for 30 minutes.

Thereafter, the reaction temperature was allowed to return to 25° C and then the mixture was stirred for 20 hours. The white precipitate which had formed in the reaction mixture was removed by suction filtration using a 11G5 glass filter, and 400 ml of ether was added to the filtrate (about 15 ml). The resultant precipitate was collected rapidly by filtration using a 11G5 glass filter, washed with 500 ml of ether and dried in a desiccator under reduced pressure to give 588 mg of the corresponding active ester.

(5) Protection of amino groups in IgG

IgG was protected with 2,3-dimethylmaleic anhydride (DMA), which was used in an amount corresponding to 30 times the number of lysine residues in IgG on the supposition that the number of lysine residues should amount to about 7 percent of the total number of all of the amino acid residues. Thus, 100 mg IgG was dissolved in 16 ml of 100 mM borate buffer (pH 8.5). 625 µl of a solution of 2,3-dimethylmaleic anhydride (200 mg/ml, in dimethylformamide) was added to the solution with ice cooling and stirring, and the resultant mixture was maintained at pH 8.5 with 0.1 M NaOH. After an hour of reaction, the reaction mixture was subjected to gel filtration in the presence of 10 mM borate buffer (pH 8.0), whereby amino-protected IgG was obtained.

Absorptiometry of the thus-obtained 2,3-dimethylmaleyl derivative of IgG (100-fold dilution) at 280 nm and 250 nm gave the following results.

Table 1

| Absorptiometry of 2,3-dimethylmaleylated IgG | | | |
|---|---|---|---|
| Sample | 280 nm | 250 nm | 280 nm/250 nm |
| Untreated IgG | 0.283 | 0.104 | 2.70 |
| Amino-protected IgG | 0.347 | 0.469 | 0.74 |

The percent recovery of the 2,3-dimethylmaleylated IgG was 95.1 percent.

(6) Conjugate preparation

The active ester obtained in the above (4) was dissolved in dimethyl sulfoxide to give a concentration of 40 mg/ml and the amino-protected IgG obtained in above (5) was dissolved in 10 mM borate buffer (pH 8.0) to give a concentration of 100 mg/ml.

A composition composed of 0.4 ml of the active ester solution, 0.1 ml of the IgG solution, 0.8 ml of dimethyl sufloxide and 1.7 ml of 50 mM phosphate buffer (pH 7.5) was subjected to reaction at 20° C for 20 hours. The reaction mixture was allowed to stand at 4° C for 30 hours in 50 mM phosphate buffer (pH 6.0)to dimethylmaleylate of the IgG and eliminate the dimethylamino groups. Then, the reaction product was purified by menas of a membrane filter (Toyo Filter Paper's UK-200) to give an adriamycin-cleaved dextran-glycin-IgG conjugate. In the preparation of the purified adriamycin-cleaved dextran-IgG conjugate, the recovery of adriamycin was 89 percent and the recovery of IgG was 96 percent.

The conjugate had a molecular weight of 230,000 and contained one molecule of dextran and 30 molecules of adriamycin per molecule of IgG antibody.

High performance gel filtration analysis (carrier: Asahipak GS-510) did not reveal the release of adriamycin-cleaved dextran conjugate from the end product conjugate.

The adriamycin-oxidized dextran content in the crude reaction mixture was not more than 5 percent and the adriamycin content therein was not more than 0.3 percent.

## EXAMPLE 2

(1) Monoclonal antibody preparation

A monoclonal antibody preparation was produced essentially by the technique described in JP-A-61-167699 using a low differentiation gastric adenoma-derived cell line (MKN-45) as an immunogen. The characteristics of the preparation are shown in Table 2 and Table 3.

Table 2

| Properties | |
|---|---|
| Globulin type | Antibody titer (enzyme antibody technique) |
| $IgG_1$ ($x$) | 1:1,000 to 1:2,000 |

Table 3: Reactivity with human-drived cancer cell lines

| Cell line | Origin | Reactivity |
|---|---|---|
| TE-1 | Esophageal carcinoma | - |
| TE-2 | " | - |
| TE-3 | " | + |
| TE-4 | " | - |
| PC-1 | Lung cancer | - |
| PC-3 | " | + |
| PC-9 | " | - |
| PC-10 | " | - |
| G-415 | Gallbladder carcinoma | - |
| HEp-2 | Laryngeal cancer | - |
| KB | Nasopharyngeal carcinoma | - |

Table 3 (cont'd)

| Cell line | Origin | Reactivity |
| --- | --- | --- |
| NRC-12 | Renal cancer | - |
| NBT-2 | Bladder cancer | - |
| J111 | Monocytic leukemia | - |
| HL-60 | Promyelocytic leukemia | - |
| COLO-210 | Colon carcinoma | - |
| COLO-320DM | " | - |
| MKN-45 | Stomach cancer | + |
| MKN-28 | " | - |
| KATO-III | " | + |
| HEK | Liver cancer | + |

The CELISA method (cf. Monoclonal Antibody, Plenum Press New York and London, 376 (1980)) was used for reactivity evaluation. The positivity (+) and negativity (-) of the reaction corresponded to the following $OD_{490}$ values in CELISA:
- : less than 0.05;
+ : not less than 0.1 but less than 0.4.

(2) Conjugate preparation

The monoclonal antibody was protected with 2,3-dimethylmaleic anhydride (DMA), which was used in an amount corresponding to 9 times the number of lysine residues in the monoclonal antibody on the supposition that the number of lysine residues should amount to about 7 percent of the total number of all of the amino acid resides. Thus, 80 mg of the monoolonal antibody was dissolved in 16 ml of 100 mM borate buffer (pH 8.5). 150 µl of a solution of 2,3-dimethylmaleic anhydride (200 mg/ml, in dimethylformamide) was added to the solution with ice cooling and stirring, and the resultant mixture was maintained at pH 8.5 with 0.1 m NaOH. After an hour of reaction, the reaction mixture was subjected to gel filtration to give an amino-protected monoclonal antibody preparation.

Absorptiometry of the thus-obtained 2,3-dimethylmaleylated monoclonal antibody (100-fold dilution) at 280 nm and 250 nm gave the following results.

Table 4

| Absorptiometry of 2,3-dimethylmaleylated monoclonal antibody | | | |
| --- | --- | --- | --- |
| Sample | 280 nm | 250 nm | 280 nm/250 nm |
| Untreated antibody | 0.160 | 0.064 | 2.54 |
| Amino-protected antibody | 0.156 | 0.236 | 0.66 |

The recovery of the 2,3-dimethylmaleylated monoclonal antibody was 97.1 percent.

A 10-mg portion of the active ester obtained in Example 1 was dissolved in 2 ml of dimethyl sulfoxide, and 0.8 ml of this solution was added to 1.2 ml of 50 mM phosphate buffer (pH 7.5) containing 5 mg of the monoclonal antibody obtained above in step (1) with stirring. After 5 minutes of reaction at 4° C, the reaction was further conducted at room temperature for 5 hours. Thereafter, deprotection was performed in phosphate buffer (pH 6.0) at 4° C for 30 hours, and the product was purified by gel filtration to give an adriamycin-cleaved dextran-glycine-monoclonal antibody conjugate. In the preparation of the purified adriamycin-cleaved dextran-monoclonal antibody preparation, the recovery of adriamycin was 93 percent and the recovery of the monoclonal antibody was 95 percent. The conjugate retained 93 percent of the antibody titer of the monoclonal antibody preparation used.

The conjugate had a molecular weight of about 220,000 and contained one molecule of dextran and 33 molecules of adriamycin per molecule of monoclonal antibody.

High performance gel filtration analysis (carrier: Asahipak GS-150) did not reveal the release of adriamycin-cleaved dextran conjugate from the end product conjugate.

The adriamycin-cleaved dextran content in the crude reaction mixture was not more than 5 percent and the adriamycin content therein was not more than 0.3 percent.

## EXAMPLE 3

(1) Oxidation of dextran

Five (5) grams of dextran (molecular weight 10,000) was weighed and dissolved in 500 ml of distilled water, 20 g of sodium periodate was added, and the oxidation reaction was performed in a dark place at room temperature for 5 hours, whereby all of the glucose residues of dextran T-10 (manufactured by Pharmacia Fine Chemicals) were oxidized. The reaction mixture was dialyzed against distilled water for 30 hours and then lyophilized to give 100% cleaved dextran.

(2) Protection with glycine

One (1) gram of the 100% cleaved dextran obtained in step (1) was weighed and dissolved in 100 ml of 50 mM phosphate buffer (pH 6.0). A solution of 480 mg of glycine in 10 ml of distilled water was added to the dextran solution. Then, 480 mg of sodium cyanoborohydride was added to the solution, the resultant mixture was stirred at 25° C for 5 hours, and the reaction mixture obtained was dialyzed against distilled water and lyophilized to give 50% cleaved dextran.

(3) Binding of adriamycin

Adriamycin was used as the anticancer substance and 2.5 mg (4.5 moles) of this was weighed and reacted with 10 mg of the cleaved dextran previously prepared (with 50% of aldehyde groups modified with glycine) in the same manner as in Example 1. The product was purified by gel filtration to give an adriamycin-cleaved dextran-glycine conjugate.

(4) Conjugate preparation

The procedure of Exmaple 2 was followed with the above intermediate conjugate to give an adriamycin-cleaved dextran-glycine-monoclonal antibody conjugate. The recovery of adriamycin was 73 percent and the recovery of the monoclonal antibody was 92 percnet.

The conjugate obtained had a molecular weight of about 200,000. The adriamycin/monoclonal antibody binding ratio was 22 and the dextran/monoclonal antibody binding ratio was 5. The relative retention of the antibody titer was 71 percent.

The molecular weight was determined by the gel filtration method (carrier: TSK gel G4000SW manufactured by Tosoh Corporation) and the adriamycin content was determined by absorptiometry at 495

nm. Glycine was determined by the Kjeldahl method.

Gel filtration (carrier: TSK gel G4000WS) of the conjugate did not demonstrate the release of adriamycin-cleaved dextran from the end product conjugate.

The adriamycin-cleaved dextran content in the crude reaction mixture was not less than 5 percent and the adriamycin content therein was not more than 1 percent.

While the invention has been described in detail and with reference to specific embodiments thereof, it will be apparent to one skilled in the art that various changes and modifications can be made therein without departing from the spirit and scope thereof.

## Claims

1. An anticancer conjugate comprising an anticancer substance coupled to a carrier via aldohexopyranose ring-cleaved dextran, wherein the aldohexopyranose ring-cleaved dextran is linked to the carrier through a carboxyl group on an amino acid protecting said dextran and an amino group on said carrier and linked to the anticancer substance through an aldehyde group on said dextran and an amino group on said anticancer substance.

2. The anticancer conjugate of Claim 1, wherein 5 to 70 percent of the aldehyde groups on the aldohexopyranose ring-cleaved dextran are protected.

3. The anticancer conjugate of Claim 1, wherein the anticancer substance is mitomycin C, adriamycin or daunomycin.

4. The anticancer conjugate of Claim 1, wherein the carrier is at least one member selected from the group consisting of gamma-globulin, cancer-specific polyclonal on monoclonal antibodies, fibronectin, transferrin and fibrinogen.

5. The anticancer conjugate of Claim 1, wherein said cleaved dextran has a molecular weight of 1,000 to 100,000.

6. The anticancer conjugate of Claim 5, wherein said cleaved dextran has a molecular weight of 10,000 to 70,000.

7. The anticancer conjugate of Claim 2, wherein 10 to 50% of the aldehyde groups on the aldohexopyranose ring-cleaved dextran are protected.

Claims for the following Contracting State: ES

1. A process for preparing an anticancer conjugate, characterized in that an anticancer substance is coupled to a carrier via aldohexopyranose ring-cleaved dextran, wherein the aldohexopyranose ring-cleaved dextran is linked to the carrier through a carboxyl group on an amino acid protecting said dextran and an amino group on said carrier, and is further linked to the anticancer substance through an aldehyde group on said dextran and an amino group on said anticancer substance.

2. A process according to claim 1, characterized in that 5 to 70 percent of the aldehyde groups on the aldohexopyranose ring-cleaved dextran are protected.

3. A process according to claim 1, characterized in that as anticancer substance mitomycin C, adriamycin or daunomycin is chosen.

4. A process according to claim 1, characterized in that a carrier is selected as at least one member from the group consisting of gamma-globulin, cancer-specific polyclonal or monoclonal antibodies, fibronectin, transferrin and fibrinogen.

5. A process according to claim 1, characterized in that said cleaved dextran has a molecular weight of 1,000 to 100,000.

6. A process according to claim 5, characterized in that said cleaved dextran has a molecular weight of 10,000 to 70,000.

7. A process according to claim 2, characterized in that 10 to 50% of the aldehyde groups on the aldohexopyranose ring-cleaved dextran are protected.